# EUROPEAN PATENT APPLICATION

(11) **EP 1 312 384 A1**
(43) Date of publication of application: **21.05.2003**
(21) Application number: 00954941.1
(22) Date of filing: 24.08.2000
(51) Int. Cl.: A61L 29/06, A61L 31/06, C09J 153/02

(54) **PROCESS FOR PRODUCING MEDICAL DEVICES**

(71) Applicant: Kuraray Co., Ltd., Kurashiki-City, Okayama 710-8622 (JP)
(72) Inventor: KIRITA, Yasuzo, Kuraray Co. Ltd., Osaka-shi, Osaka 530-8611 (JP); NAKASHIMA, Toshihide, Kuraray Co. Ltd., Kurashiki-shi, Okayama 710-8622 (JP); UTAGAWA, Nobuyuki, Kuraray Co. Ltd., Kurashiki-shi, Okayama 710-8622 (JP); WADA, Koichi, Kuraray Co. Ltd., Kashima-gun, Ibaraki 314-0197 (JP); JOGO, Yosuke, Kuraray Co. Ltd., Kashima-gun, Ibaraki 314-0197 (JP); MAEDA, Mizuho, Kuraray Co. Ltd., Kashima-gun, Ibaraki 314-0197 (JP); INAI, Koji, Kuraray Co. Ltd., Kirashiki-shi, Okayama 710-8622 (JP)
(74) Representative: HOFFMANN - EITLE
(86) International application number: JP0005656
(87) International publication number: WO02015953

(57) **Abstract**

There are provided a process for producing a medical device comprising adhering parts for a medical device comprising an olefinic resin and/or a thermoplastic elastomer with an adhesive comprising a solution prepared by dissolving in an organic solvent a block copolymer or a hydrogenated product thereof, the block copolymer comprising 10 to 40% by weight of a polymer block comprising an aromatic vinyl compound unit, and 90 to 60% by weight of at least one polymer block selected from the group consisting of a polyisoprene block, an isoprene-butadiene polymer block, and a polybutadiene block; a medical device obtainable by the process; an adhesive used for the production; and the like.

## Description

### TECHNICAL FIELD

The present invention relates to a process for producing a medical device, and a medical device obtainable by the process.

The process for producing a medical device of the present invention gives a medical device with excellent operability and strong adhesive strength at an adhering part, and without lowering adhesive strength when subjected to sterilization. The medial device is highly safe because a substance elutes a little from the medical device including the adhering part into blood or body fluid, and there is almost no substance eluted into physiological saline to be used during treatment, ethanol to be used for disinfection or the like.

### BACKGROUND ART

Although medical devices have been produced by using an olefinic resin and a resin composition comprising an olefinic resin, it is difficult to adhere these materials. In many cases, adhesion is usually carried out by subjecting a resin surface to corona treatment, plasma treatment, ozonation, primer treatment as a chemical treatment, or the like, and thereafter adhering the treated surfaces with an adhesive. As an adhesive therefor, cyanoacrylate-based adhesives, epoxy-based adhesives, and synthetic rubber-based adhesives have been made commercially available.

A primer is required for adhering an olefinic resin with the cyanoacrylate-based adhesive, so that a step comprising applying the primer to an adhering surface, thereafter applying an adhesive thereto, and adhering the surfaces in a short period of time must be taken, thereby making its operability poor. Further, the cyanoacrylate-based adhesive itself cures and becomes unusable, so that the handling of the adhesive requires much care.

A primer is required for adhering an olefinic resin with the epoxy-based adhesive, so that a step comprising treating an adhering surface with a primer, thereafter applying an epoxy-based adhesive of a mixture of two liquids thereto, and adhering the surfaces must be taken. Since the adhesive of a mixture of two liquids undesirably cures in 10 minutes or so, the liquids must be mixed for each application, thereby giving poor operability for use in production on an industrial scale.

As to the synthetic rubber-based adhesive, adhesion must be carried out by applying an adhesive to an adhering surface, thereafter drying for about 10 minutes, and thereafter strongly pressing together the adhering surfaces, thereby making its operability poor. In addition, in many cases, these adhesives contain an additive dissolved in body fluid or blood, thereby making it inappropriate as an adhesive for production of medical devices.

Japanese Patent Laid-Open No. Hei 2-153986 has proposed that a thermoplastic elastomer is used as a main raw material for a hot melt adhesive. Since this adhesive does not contain water or a solvent, a drying step is not required. However, a large-scaled specialized device so-called "applicator" is required for melting and applying the resin.

Japanese Patent Laid-Open No. Hei 11-50031 has proposed an adhesive comprising a styrene block copolymer rubber and a tackifying resin, a low-molecular weight α-olefinic polymer and a solvent as essential components, which is used for adhering a polyolefinic resin. Although this adhesive has improved operability, its adhesive strength is insufficient, and a substance eluted into blood or ethanol exists, thereby making it inappropriate as an adhesive for producing a medical device.

Further, there may be some cases where the adhesive strength varies depending upon sterilization procedure (ethylene oxide gas sterilization, autoclaving, gamma ray sterilization, electron beam sterilization, or the like) conducted after the production of the medical device, so that the medical device cannot be used clinically.

Soft vinyl chloride resins have been widely used as materials for medical devices, and the materials contain a plasticizer in a large amount, so that its elution into blood or body fluid has been recognized as a problem.

In order to solve this problem, there has been proposed in Japanese Patent Laid-Open No. Hei 10-67894 a resin composition comprising a polypropylene-based resin and a hydrogenated block copolymer comprising a polymer block comprising an aromatic vinyl compound and a polymer block comprising isoprene and/or butadiene having a content of vinyl bonds within a specified range. This resin composition has flexibility and transparency, has properties substitutable for a soft vinyl chloride resin, and does not contain a substance such as a plasticizer which elutes into blood or body fluid, or further elutes into ethanol. Therefore, the resin composition has been expected as a highly safe material for medical device. In addition, the olefinic resin is more rigid than the above-mentioned resin composition in its properties, and thus has a limited range of use. Like the resin composition, however, there is little eluting substance, so that it is highly safe material.

On the other hand, in order to produce a medical device, in most cases, adhesion between parts is required. As the properties of the adhesive, there have been required that i) the adhesive strength is sufficient; ii) there is no substance eluted into blood, body fluid, physiological saline or ethanol; iii) the adhesive strength is not lowered even in blood, body fluid, physiological saline or ethanol; iv) the adhesive strength is not lowered by sterilization; and v) the operability is excellent. However, there have not yet been found an adhesive which can adhere the above-mentioned resin composition and the olefinic resin and satisfy the above-mentioned properties.

### DISCLOSURE OF INVENTION

An object of the present invention is to provide a process for producing a medical device comprising adhering parts of the medical device comprising an olefinic resin and/or a thermoplastic elastomer by using an adhesive satisfying items i) to v) above, and a medical device obtainable by the process.

Another object of the present invention is to provide the above-mentioned adhesive.

According to the present invention, the above-mentioned object can be achieved by providing a process for producing a medical device, characterized by adhering parts for a medical device comprising an olefinic resin (I) and/or a thermoplastic elastomer (II) with an adhesive comprising a solution prepared by dissolving in an organic solvent a block copolymer or a hydrogenated product thereof, the block copolymer comprising 10 to 40% by weight of a polymer block (A) comprising an aromatic vinyl compound unit; and 90 to 60% by weight of at least one polymer block (B) selected from the group consisting of a polyisoprene block (b1), an isoprene-butadiene polymer block (b2), and a polybutadiene block (b3), and a medical device obtainable by the process.

Also, according to the present invention, the above-mentioned object can be achieved by providing an adhesive comprising a solution prepared by dissolving in an organic solvent a block copolymer or a hydrogenated product thereof, the block copolymer comprising 10 to 40% by weight of a polymer block (A) comprising an aromatic vinyl compound unit; and 90 to 60% by weight of at least one polymer block (B1) selected from the group consisting of a polyisoprene block (b11) having a content of 1,2-bond and 3,4-bond of 10% by mol or more, an isoprene-butadiene polymer block (b21) comprising 5 to 95% by weight of isoprene unit and 95 to 5% by weight of butadiene unit and having a content of 1,2-bond and 3,4-bond of 20% by mol or more, and a polybutadiene block (b31) having a content of 1,2-bond of 30% by mol or more.

### BEST MODE FOR CARRYING OUT THE INVENTION

The aromatic vinyl compound which constitutes an aromatic vinyl compound unit in the polymer block (A) of the above-mentioned block copolymer includes styrene, α-methylstyrene, 1-vinylnaphthalene, 3-methylstyrene, 4-propylstyrene, 4-cyclohexylstyrene, 4-dodecylstyrene, 2-ethyl-4-benzylstyrene, 4-(phenylbutyl)styrene, and the like. Among them, styrene is preferable. The number-average molecular weight of the polymer block (A) comprising the aromatic vinyl compound units is not limited to specified ones, and the number-average molecular weight is preferably within the range of 2500 to 20000.

The polymer block (B) is at least one polymer block selected from the group consisting of a polyisoprene block (b1), an isoprene-butadiene polymer block (b2) and a polybutadiene block (b3).

The content of 1,2-bond and 3,4-bond (content of vinyl bonds) in the polyisoprene block (b1) is preferably 10% by mol or more, more preferably 40% by mol or more, especially preferably 50% by mol or more, from the viewpoint of increasing the adhesive strength. The number-average molecular weight of the polyisoprene block (b1) is not limited to specified ones, and the number-average molecular weight is preferably within the range of 10000 to 200000.

The isoprene-butadiene polymer block (b2) preferably comprises 5 to 95% by weight of isoprene unit and 95 to 5% by weight of butadiene unit. The content of 1,2-bond and 3,4-bond (content of vinyl bonds) in the block is preferably 20% by mol or more, more preferably 40% by mol or more, from the viewpoint of increasing the adhesive strength. The polymerization form of the isoprene and butadiene in the isoprene-butadiene polymer block (b2) is not limited to specified ones, and the polymerization form may be any of random, block, tapered forms, and the like. In addition, the number-average molecular weight of the isoprene-butadiene polymer block (b2) is not limited to specified ones, and the number-average molecular weight is preferably within the range of 10000 to 200000.

The content of 1,2-bond (content of vinyl bonds) in the polybutadiene block (b3) is preferably 30% by mol or more, more preferably 45% by mol or more, especially preferably 60% by mol or more, from the viewpoint of increasing the adhesive strength. The number-average molecular weight of the polybutadiene block (b3) is not limited to specified ones, and the number-average molecular weight is preferably within the range of 10000 to 200000.

The content of the polymer block (A) in the block copolymer in the present invention is within the range of from 10 to 40% by weight, preferably within the range of from 15 to 30% by weight. The content of the polymer block (B) in the above-mentioned block copolymer is from 90 to 60% by weight, preferably within the range of from 85 to 70% by weight. When the content of the polymer block (A) is less than 10% by weight, the adhesive strength of the adhesive is lowered, and when the content exceeds 40% by weight, the viscosity of the resulting adhesive becomes high, so that the coatability of the adhesive becomes poor, and the adhesive strength becomes insufficient.

Further, preferably 70% by mol or more, more preferably 85% by mol or more, of the carbon-carbon double bonds in the polymer block (B) are hydrogenated, from the viewpoint of increasing the weathering resistance.

The bonding form of each polymer block in the above-mentioned block copolymer is not limited to specified ones, and may be any of linear form, branched form and a given combination of these. Concrete examples of the molecular structure of the block copolymer include A-(B-A)ₖ, (A-B)ₙ, and the like, wherein A and B are the polymer block (A) and the polymer block (B), respectively, and k and n are each an integer of 1 or more. In addition, the block copolymer may have a star-like form obtained by using a coupling agent such as divinylbenzene, a tin compound or a silane compound, such as (A-B)ₘX,
wherein m is an integer of 2 or more, and X is a residue of a coupling agent. The bonding form of the polymer block is preferably A-B-A.

As the block copolymer, those having various molecular structures mentioned above may be used alone, or in admixture of two or more different molecular structures, such as a mixture of triblock-type and diblock-type. The number-average molecular weight of the block copolymer is preferably within the range of 30000 to 300000.

The above-mentioned block copolymer can be prepared, for instance, by the following processes (i) to (iii) or the like:
(i) a process comprising polymerizing an aromatic vinyl compound using an alkyllithium compound as an initiator, and thereafter sequentially polymerizing a conjugated diene compound (isoprene, butadiene) and an aromatic vinyl compound;
(ii) a process comprising polymerizing an aromatic vinyl compound and subsequently a conjugated diene compound, to give a block copolymer, and coupling the resulting block copolymer with a coupling agent; and
(iii) a process comprising polymerizing a conjugated diene compound using dilithium compound as an initiator, and thereafter sequentially polymerizing an aromatic vinyl compound.

As the alkyllithium compound in the above-mentioned process, there can be used a compound having an alkyl group having 1 to 10 carbon atoms, among which methyllithium, ethyllithium, pentyllithium, n-butyllithium, s-butyllithium, and t-butyllithium are preferable.

The coupling agent includes, for instance, halogenated compounds such as dichloromethane, dibromomethane, dichloroethane, dibromoethane, dibromobenzene, tin tetrachloride; ester compounds such as phenyl benzoate and ethyl acetate; divinylbenzene; various silane compounds; and the like.

The dilithium compound includes, for instance, naphthalenedilithium, dilithiohexylbenzene, and the like.

The amount of the above-mentioned initiator or coupling agent used is properly determined depending upon the molecular weight of the desired block copolymer. Usually, the initiator is used in an amount within the range of from 0.01 to 0.2 parts by weight, and the coupling agent is used in an amount within the range of from 0.04 to 0.8 parts by weight, based on 100 parts by weight of the entire monomers to be used in the polymerization.

In addition, the content of vinyl bonds in the polyisoprene block (b1), the isoprene-butadiene polymer block (b2) and the polybutadiene block (b3) can be controlled by using a Lewis base as a co-catalyst during the polymerization. The Lewis base includes, for instance, ethers such as dimethyl ether, diethyl ether and tetrahydrofuran; glycol ethers such as ethylene glycol dimethyl ether and diethylene glycol dimethyl ether; amine compounds such as triethylamine, N,N,N',N'-tetramethylethylenediamine and N-methylmorpholine; and the like. The amount of the Lewis base used is preferably within the range of from 0.1 to 1000 mol per 1 mol of lithium atoms in the polymerization initiator.

During the polymerization, an organic solvent which is inert against the polymerization initiator is used as a solvent. As the solvent, it is preferable to use an aliphatic hydrocarbon having 6 to 12 carbon atoms such as hexane or heptane; an alicyclic hydrocarbon such as cyclohexane or methylcyclohexane; or an aromatic hydrocarbon such as benzene.

When the block copolymer is hydrogenated, there can be employed, for instance, a known process such as a process comprising reacting hydrogen in a molecular state with a block copolymer dissolved in an inert solvent for the reaction in the presence of a hydrogenation catalyst. As the hydrogenation catalyst used herein, there can be used a heterogeneous catalyst in which a metal such as nickel, platinum, palladium, ruthenium or rhodium is supported by a substrate such as carbon, alumina or diatomaceous earth; a Ziegler-based catalyst comprising a combination of an organometallic compound of a metal of Group VIII such as nickel or cobalt, with an organoaluminum compound such as triethylaluminum or triisobutylaluminum, an organolithium compound or the like; a metallocene-based catalyst comprising a combination of a bis(cyclopentadienyl) compound of a transition metal such as titanium, zirconium or hafnium, with an organometallic compound of lithium, sodium, potassium, aluminum, zinc, magnesium or the like; or the like. The hydrogenation is carried out usually at a hydrogen pressure within the range of a normal pressure to 200 kg/cm² and a reaction temperature within the range of ambient temperature to 250°C. The reaction time is usually from 0.1 to 100 hours. The hydrogenated block copolymer formed by the hydrogenation is obtained by (i) solidifying the reaction mixture solution with methanol or the like, and thereafter heating or drying under reduced pressure; or (ii) subjecting the reaction solution to a so-called "steam-peel" in which the reaction solution is poured into boiling water, and the solvent is removed by azeotropic treatment, and thereafter heating or drying under reduced pressure.

An adhesive usable in the present invention can be prepared by dissolving the block copolymer or a hydrogenated product thereof thus obtained in an organic solvent, to give a solution. The above-mentioned organic solvent includes toluene, xylene, tetrahydrofuran, n-hexane, cyclohexanone, cyclohexane, methylcyclohexane, isoamyl acetate, n-heptane, benzene, and the like. Toluene, xylene, tetrahydrofuran, cyclohexanone, cyclohexane and methylcyclohexane are preferable, and toluene and tetrahydrofuran are more preferable, from the viewpoint of easily dissolving the block copolymer or a hydrogenated product thereof in the organic solvent.

The concentration of the block copolymer or a hydrogenated product thereof in the solution as the above-mentioned adhesive may depend upon their solubility against an organic solvent. The concentration is preferably within the range of 3 to 30% by weight, more preferably within the range of 10 to 20% by weight, from the viewpoint of the coating operability of the adhesive.

It is preferable that an adhesive is a solution prepared by dissolving in an organic solvent a block copolymer comprising 10 to 40% by weight of a polymer block (A) comprising an aromatic vinyl compound unit; and 90 to 60% by weight of at least one polymer block (B1) selected from the group consisting of a polyisoprene block (b11) having a content of 1,2-bond and 3,4-bond of 10% by mol or more, an isoprene-butadiene polymer block (b21) comprising 5 to 95% by weight of isoprene unit and 95 to 5% by weight of butadiene unit and having a content of 1,2-bond and 3,4-bond of 20% by mol or more, and a polybutadiene block (b31) having a content of 1,2-bond of 30% by mol or more.

The above-mentioned adhesive has excellent adhesive strength against various resins, and especially high adhesive strength against an olefinic resin and/or a thermoplastic elastomer (especially styrene/diene-based block copolymer), and is useful as an adhesive for a medical device such as blood bag, blood tube, infusion solution bag, infusion solution tube, blood circuit, or catheter; an adhesive for hygiene materials such as sanitary napkin and paper diaper; or an adhesive for a medical product such as operating gown or disposable sheets for hospitals.

Further, in the above-mentioned adhesive, since the organic solvent contained in the adhesive evaporates during adhesion and almost does not exist in the adhering part, there is little substance eluted into blood or body fluid, so that it is suitable as an adhesive for a medical device. In addition, since the adhesive is durable even at a temperature of autoclaving (121°C) and its adhesive strength is not lowered by sterilization treatment, it is suitable as an adhesive usable in a medical device requiring sterilization.

By adhering parts such as a tube, a connector, a drip chamber, an air chamber, a pillow, a cuff and a sheet using the above-mentioned adhesive, the medical device can be produced. As the process of adhesion, there can be employed a process comprising applying an adhesive to an adhering part, and adhering parts; a process comprising immersing an adhering part in an adhesive, and thereafter adhering parts; and the like. In this case, the process comprising immersing an adhering part in an adhesive, and thereafter adhering parts is preferable, from the viewpoints of the operability and the adhesive strength. The adhesive may be applied to one or both of the adhering parts, and the adhesion can be carried out by bringing the adhering surfaces together immediately after applying the adhesive, or after drying for several minutes. In addition, after the adhesion, the adhered parts may be allowed to stand at room temperature until a sufficient adhesive strength is obtained. It is preferable that the parts are heat-treated to an extent such that the medical device is not deformed. Its heating temperature is preferably from 60° to 100°C.

The materials for the above-mentioned parts are preferably the olefinic resin (I) and/or the thermoplastic elastomer (II). The material is more preferably a composition comprising 10 to 90% by weight of the olefinic resin and 90 to 10% by weight of the thermoplastic elastomer, still more preferably a composition comprising 20 to 80% by weight of the olefinic resin and 80 to 20% by weight of the thermoplastic elastomer, especially preferably a composition comprising 50 to 20% by weight of the olefinic resin and 50 to 80% by weight of the thermoplastic elastomer, from the viewpoints of the transparency, the heat setting property and the heating processability. In addition, as the materials for above-mentioned parts, there can be used those which can be generally used as materials for medical devices such as resins other than the olefinic resin (I) and the thermoplastic elastomer (II), metals, and glass. The term "heat setting property" refers to a property in which a resin composition is fixed into a given shape by subjecting the resin composition to extrusion molding and thereafter repeating heating and cooling, so that its initial shape is not recovered easily. In addition, as the above-mentioned transparency, those having Haze value of 20% or less are preferable, when the resin composition is made into a sheet having a thickness of 1 mm.

The olefinic resin (I) includes polyethylenes such as high-density polyethylene and low-density polyethylene, polypropylene, polymethylpentene-1 and a resin comprising these resins as a main component. Among them, polypropylene is preferable. The polypropylene may be a homopolymer of propylene, or a copolymer containing copolymer components such as ethylene, and the polypropylene may be random polypropylene or block polypropylene. These polypropylenes may be used alone or in admixture of two or more kinds. As the melt viscosity of the polypropylene, those having a melt flow rate (MFR) within the range of from 0.1 to 500 are preferable, more preferably within the range of from 2 to 200, as determined by the process according to ASTMD-1238 at 230°C and a load of 2160 g.

The thermoplastic elastomer (II) includes aromatic vinyl-based thermoplastic elastomers, polyolefinic thermoplastic elastomers, polydiene-based thermoplastic elastomers, chlorine-based thermoplastic elastomers,
polyurethane-based thermoplastic elastomers, polyester-based thermoplastic elastomers, fluorine-based thermoplastic elastomers, and the like. Examples thereof include styrene-butadiene-styrene block copolymers, styrene-isoprene-styrene block copolymers, styrene-ethylene·butylene-styrene block copolymers, styrene-ethylene·propylene-styrene block copolymers, styrene-butadiene random copolymers, 1,2-polybutadienes, ethylene-propylene rubbers, polyisoprene rubbers, and the like. Among them, the aromatic vinyl-based thermoplastic elastomers and the polyolefinic thermoplastic elastomers are preferable, because they have excellent compatibility with the olefinic resin, and the aromatic vinyl-based thermoplastic elastomers are more preferable. The aromatic vinyl-based thermoplastic elastomer includes random copolymers or block copolymers comprising an aromatic vinyl compound unit and a conjugated diene unit or the like, among which the block copolymer is preferable, more preferably at least one block copolymer selected from the group consisting-of a block copolymer and a hydrogenated product thereof, the block copolymer comprising a polymer block (C) comprising an aromatic vinyl compound unit and a polymer block (D) comprising a conjugated diene unit, and having a content of the aromatic vinyl compound unit of from 10 to 40% by weight.

The compound which constitutes the aromatic vinyl compound unit in the polymer block (C) includes styrene, α-methylstyrene, 1-vinylnaphthalene, 3-methylstyrene, 4-propylstyrene, 4-cyclohexylstyrene, 4-dodecylstyrene,
2-ethyl-4-benzylstyrene, 4-(phenylbutyl)styrene, and the like. Among them, styrene is preferable. The number-average molecular weight of the polymer block (C) is not limited to specified ones, and the number-average molecular weight is preferably within the range of 2500 to 20000.

The conjugated diene in the polymer block (D) includes isoprene, butadiene, hexadiene, 2,3-dimethyl-1,3-butadiene, 1,3-pentadiene, and the like. Among them, isoprene and butadiene are preferable.

The polymer block (D) is preferably at least one polymer block selected from the group consisting of a polyisoprene block (d1), an isoprene-butadiene polymer block (d2) and a polybutadiene block (d3).

The content of 1,2-bond and 3,4-bond (content of vinyl bonds) in the polyisoprene block (d1) is preferably 10% by mol or more, more preferably from 40 to 75% by mol, from the viewpoints of increasing the transparency and appropriately lowering the glass transition temperature (Tg).

In the isoprene-butadiene polymer block (d2), the weight ratio of isoprene to butadiene is preferably within the range of from 5/95 to 95/5, and the content of 1,2-bond and 3,4-bond (content of vinyl bonds) is preferably 20% by mol or more, more preferably from 60 to 85% by mol, from the viewpoints of increasing the transparency and appropriately lowering the glass transition temperature (Tg). In addition, the polymerization form of the isoprene and butadiene is not limited to specified ones, and the polymerization form may be any of random, block, tapered forms, and the like.

The content of 1,2-bond (vinyl bond) in the polybutadiene block (d3) is preferably 30% by mol or more, more preferably 60% by mol or more, from the viewpoint of the transparency.

Preferably 70% by mol or more, more preferably 85% by mol or more, of the carbon-carbon double bonds in the polyisoprene block (d1), the isoprene-butadiene polymer block (d2) and the polybutadiene block (d3) are hydrogenated. When the hydrogenation ratio is 70% by mol or more, the weathering resistance and the heat resistance are improved, so that the deterioration of the resulting medical device upon use can be prevented, and that its compatibility with the polypropylene-based resin is increased, thereby improving the transparency.

The bonding form of each polymer block in the above-mentioned block copolymer is not limited to specified ones, and may be any of linear form, branched form and a given combination of these. Concrete examples of the molecular structure of the block copolymer include C-(D-C)ₖ, (C-D)ₙ, and the like, wherein C and D are the polymer block (C) and the polymer block (D), respectively, and k and n are each an integer of 1 or more. In addition, the block copolymer may have a star-like form obtained by using a coupling agent such as divinylbenzene, a tin compound or a silane compound, such as (C-D)ₘX,
wherein m is an integer of 2 or more, and X is a residue of a coupling agent. The bonding form of the polymer block is preferably C-D-C.

The number-average molecular weight of the polyisoprene block (d1), the isoprene-butadiene polymer block (d2) and the polybutadiene block (d3) is not limited to specified ones, and the number-average molecular weight is preferably within the range of 10000 to 200000.

The medical device produced from parts using the above-mentioned olefinic resin (I) and/or thermoplastic elastomer (II) as a material is especially suitable as a tracheal catheter or vessel catheter. In addition, an additive can be added to the olefinic resin (I) and/or thermoplastic elastomer (II), within the range so as not to impair the function as and safety for a medical device. The additive includes an antioxidant, an ultraviolet absorbent, a photostabilizer, a colorant, a crystalline nucleus agent, an inorganic filler such as talc, cross-linked beads, and the like. It is preferable that the amount of these additives used is usually 5 parts by weight or less, based on 100 parts by weight of the olefinic resin (I) and/or thermoplastic elastomer (II). In addition, a tackifying resin, including a hydrogenated resin such as a hydrogenated coumarone-indene resin, a hydrogenated rosin-based resin, a hydrogenated terpene resin, or an alicyclic hydrogenated petroleum resin can be added. It is preferable that the amount of these tackifying resins used is less than 200 parts by weight, based on 100 parts by weight of the olefinic resin (I) and/or thermoplastic elastomer (II). Also, an oil such as a petroleum-based hydrocarbon can be added.

In addition, there can be formulated to the olefinic resin (I) and/or thermoplastic elastomer (II), for instance, another polymer such as an ethylenevinyl acetate copolymer, an ethylene-methacrylic acid copolymer, an ethylene-acrylic acid copolymer, an ionomer thereof, or an ethylene-ethyl acrylate copolymer, within the range so as not to hinder the object of the invention. Also, the olefinic resin (I) and/or thermoplastic elastomer (II) used in the present invention can be cross-linked by a usual cross-linking process using a peroxide or the like as desired.

The part constituting the medical device can be produced by kneading an olefinic resin (I) and/or thermoplastic elastomer (II) with a kneading device such as a kneader, a Banbury mixer or a roller to give a resin composition as desired, and molding the resin composition by a process such as extrusion molding, injection molding, or press molding.

Next, as one embodiment of the medical device of the present invention, a process for producing a tracheal catheter will be explained.

When a tracheal catheter is inserted into the trachea, the catheter is required to be not only capable of working into a flexible curve of an arc-shape, but also drastically bending the catheter to a flex angle of nearly 180°, from the viewpoint of suppressing damage on the pharynx, the trachea, or the vocal cord.

In order to work a tube comprising the olefinic resin (I) and/or thermoplastic elastomer (II) into a curve, there have been employed a process comprising fitting a tube into a grooved mold, heating and cooling the tube without causing kinking, thereafter placing the tube in a grooved mold with increased curvature, and again repeating heating and cooling the tube; a process comprising placing a tube into a so-called corrugated tube in place of the grooved mold, and heating and cooling the tube without causing kinking, and gradually increasing the curvature of the corrugated tube; a process comprising winding helically on to a tube with a wire-like object such as a wire or a metal plate with a narrow width to bend the tube, and repeating heating and cooling, thereby gradually increasing the flex angle of the tube; and the like. Among them, the process of winding helically on to a tube with a wire-like object is more excellent in the operability, the reproducibility, and the working precision. Concretely, a wire having a diameter of 0.5 mm to 2.5 mm is wound helically on to the tube, and heating and cooling the tube are repeated, whereby the curvature of the tube can be gradually increased. After heat setting, a shape as curved is retained even when a wire or the like wound on to the tube is removed, and the original shape of the tube is not easily recovered. The heating may be carried out at a temperature of 70° to 150°C, and the cooling may be carried out by cooling to room temperature with water or the like. By repeating the above operations, the curvature is increased.

A cuff is adhered to the curved tube thus produced, and a branched tube to which a pilot cuff and a connector are previously attached is further adhered to the tube. The above-mentioned adhesive is used for adhesion. Finally, a connector for connecting the tube with a respirator is inserted thereinto, to give a tracheal catheter. The materials for the connector, the cuff, the pilot cuff, and the branched tube are preferably the olefinic resin (I) and/or thermoplastic elastomer (II), more preferably a resin composition comprising 20 to 80% by weight of the olefinic resin (I) and 80 to 20% by weight of the thermoplastic elastomer (II).

Next, as another embodiment of the medical device of the present invention, a process for producing a vessel catheter will be explained.

The catheter inserted into the blood vessel is led to one-way at a branched blood vessel site by using a guide wire. Therefore, it is preferable that an end part near the tip end of the catheter is previously curved. Since a tube having a narrow diameter and a thin-walled tube are generally used as a vessel catheter, the vessel catheter can be worked by using a mold having a groove with a desired flex angle. In-this case, the molding is carried out at a temperature of from 90° to 150°C, preferably from 100° to 135°C.

A connector for connecting the resulting curved tube comprising the olefinic resin (I) and/or thermoplastic elastomer (II) thus produced with an infusion solution tube or the like is connected with the curved tube by using the above-mentioned adhesive, to give a vessel catheter. The material for the connector is preferably the olefinic resin (I) and/or thermoplastic elastomer (II), more preferably a resin composition comprising 20 to 80% by weight of the olefinic resin (I) and 80 to 20% by weight of the thermoplastic elastomer (II).

Since the catheter as described above is inserted into the trachea or the blood vessel, the damage on the trachea or the blood vessel must be suppressed. Therefore, it is required that a tip end of the tube is subjected to chamfer treatment. In order to subject the tip end of the tube to a chamfer treatment, there have been known a process comprising cutting a tip end of a tube, and immersing a cut edge in an organic solvent, thereby chamfering the cut edge; a process comprising heating a cut edge with hot air or incandescent lamp, thereby chamfering the cut edge; or the like. However, in the process using an organic solvent, since the tube is dissolved in the organic solvent, the tube itself is slimmed even though the cut edge can be chamfered, and the tube becomes opaque, so that its transparency is impaired. In addition, since the inner diameter is small, the solvent penetrates into the internal of the tube by the capillary phenomenon, thereby clogging the tube. In the process taking an advantage of heating the cut edge with hot air or incandescent lamp, a time period of 10 minutes or more is required until reaching the temperature at which the cut edge can be chamfered, namely the tube starts to melt, thereby making its operability poor.

In order to eliminate the above-mentioned defects, it is preferable to employ a chamfering process comprising cutting a tip end of a catheter, preferably at an angle of 45°, and pressing the tip end to holes provided in a mold at a temperature of 100° to 135°C. This process is especially excellent from the aspects of operability, reproducibility, and working precision. As the shape of the hole on the mold, there may be employed hemispherical shape, conical shape, or the like, and parabolic shape is preferable. When the molding temperature is too high, the fusion of a tube to a mold is caused, and when the molding temperature is too low, the pressing time period becomes too long, making it unpractical. Therefore, although the molding temperature and the pressing time depend upon the thickness of a catheter, the molding temperature is preferably within the range of from 100° to 135°C, and the pressing time is preferably 30 seconds or so, from the viewpoint of the operability.

The above-mentioned adhesive can be applied for the production of various medical devices other than those catheters mentioned above. For instance, a blood circuit for hemodialysis comprises a tube, a connector, an air chamber, a pillow and the like, and the blood circuit can be produced by adhering each of the parts. In addition, a blood bag comprises a main body bag made of sheets pasted together, a tube and a connector, and the blood bag is produced by adhering each of the parts. The material for each part mentioned above is preferably the olefinic resin (I) and/or thermoplastic elastomer (II), and other resin such as a polycarbonate, a metal or the like can be partially used without any problem. In addition, as to the adhesion, there is no problem even if heat-fusion or the like is partially employed.

The medical device obtainable by the present invention has a high strength in the adhesive part, such that the adhesive strength is not lowered in blood, body fluid, physiological saline and ethanol even after sterilization. Further, the medical device is highly safe because there is no substance eluted into blood, body fluid, physiological saline and ethanol. In other words, a highly safe medical method can be performed when the medical device of the present invention is applied to a human body. Therefore, according to the present invention, there is provided a medical method, comprising the step of applying the medical device of the present invention to a human body.

### EXAMPLES

The present invention will be explained by means of the working examples, without intending to limit the present invention thereto.

### Preparation Examples 1 and 2: Preparation of Block Copolymers

In a dried, nitrogen-exchanged pressure vessel, styrene was polymerized at 60°C using cyclohexane as a solvent and s-butyllithium as a polymerization initiator. Thereafter, tetrahydrofuran was added thereto as a Lewis base, and subsequently, isoprene and styrene were sequentially polymerized, to give two kinds of block copolymers of styrene-isoprene-styrene form. The content of styrene, the number-average molecular weight, and the content of vinyl bonds of the resulting block copolymer are shown in the following Table 1.

### Preparation Example 3: Preparation of Hydrogenated Block Copolymer

The block copolymer of styrene-isoprene-styrene form obtained in Preparation Example 2 was hydrogenated in cyclohexane at a hydrogen pressure of 20 kg/cm² in the presence of palladium-carbon as a catalyst, to give a hydrogenated block copolymer. The content of styrene, the number-average molecular weight, the content of vinyl bonds, and the hydrogenation ratio of the resulting hydrogenated block copolymer are shown in the following Table 1.

### Preparation Example 4: Preparation of Hydrogenated Block Copolymer

Styrene, a mixture of isoprene and butadiene [isoprene/butadiene = 60/40 (weight ratio)], and styrene were sequentially polymerized in a cyclohexane solvent by using s-butyllithium and tetrahydrofuran in the same manner as those in Preparation Examples 1 and 2, to give a block copolymer of styrene-(isoprene/butadiene)-styrene form. The resulting block copolymer was hydrogenated in the same manner as that in Preparation Example 3, to give a hydrogenated block copolymer. The content of styrene, the number-average molecular weight, the content of vinyl bonds, and the hydrogenation ratio of the resulting hydrogenated block copolymer are shown in the following Table 1.

### Preparation Examples 5 and 6: Preparation of Hydrogenated Block Copolymers

In a dried, nitrogen-exchanged pressure vessel, styrene was polymerized at 60°C using cyclohexane as a solvent and s-butyllithium as a polymerization initiator. Thereafter, isoprene and styrene were sequentially polymerized, to give a block copolymer of styrene-isoprene-styrene form. Further, the block copolymer was hydrogenated in cyclohexane at a hydrogen pressure of 20 kg/cm² in the presence of palladium-carbon as a catalyst, to give a hydrogenated block copolymer. The content of styrene, the number-average molecular weight, the content of vinyl bonds, and the hydrogenation ratio of the resulting hydrogenated block copolymer are shown in the following Table 1.

The content of styrene, the number-average molecular weight, the content of vinyl bonds, and the hydrogenation ratio mentioned above were determined by the following methods.
1) Content of styrene: The content of styrene was calculated from the weight of each monomer component used in polymerization.
2) Number-average molecular weight: A number-average molecular weight was determined as calculated in terms of polystyrene by GPC determination.
3) Content of vinyl bonds: The block copolymer before hydrogenation was dissolved in chloroform deuteride (CDCl₃), and its ¹H-NMR spectrum was determined. The content of vinyl bonds was calculated from the size of the peak corresponding to 1,2-bond or 3,4-bond.
4) Hydrogenation ratio: The iodide values of the block copolymer before and after hydrogenation were determined, and the ratio was calculated from the found values.

**Table 1**

| Preparation Example | Molecular Structure Before Hydrogenation (Note) | Content of Styrene (% by wt.) | Number - Average Molecular Weight (× 10⁴) | Content of Vinyl Bonds (% by mol) | Hydrogenation Ratio (%) |
|---|---|---|---|---|---|
| Prep. Ex. 1 | A-B-A | 20 | 8.5 | 70 | 0 |
| Prep. Ex. 2 | A-B-A | 20 | 8.9 | 55 | 0 |
| Prep. Ex. 3 | A-B-A | 20 | 8.8 | 55 | 93 |
| Prep. Ex. 4 | A-C-A | 15 | 6.4 | 60 | 95 |
| Prep. Ex. 5 | A-B-A | 20 | 19.2 | 7 | 98 |
| Prep. Ex. 6 | A-B-A | 65 | 6.4 | 7 | 97 |

| | | | | | |
|---|---|---|---|---|---|
| (Note) A: Polystyrene block B: Polyisoprene block C: Poly(isoprene/butadiene) block | | | | | |

### Preparation Examples 7 to 10

Each of the block copolymers obtained in Preparation Examples 2 to 5 was prepared into a toluene solution, the concentration of which was 20% by weight, to give an adhesive.

### Preparation Examples 11 to 13

Each of the block copolymers obtained in Preparation Examples 1 to 3 was prepared into a tetrahydrofuran solution, the concentration of which was 20% by weight, to give an adhesive.

### Comparative Example 1

The block copolymer obtained in Preparation Example 6 was prepared into a toluene solution, the concentration of which was 20% by weight, to give an adhesive.

### Test Example 1: Changes in Peel Strength of Adhesion Before and After Sterilization

Two sheets of a polypropylene sheet (thickness: 100 µm) having a rectangular shape of 1 cm x 2 cm were cut out, and 15 µl of the adhesive obtained in Preparation Examples 7 to 9 and 11 to 13 and Comparative Example 1 was applied to a half of a side (1 cm²), and the other half was covered with a Teflon sheet having a thickness of 100 µm. Thereafter, the two sheets were pasted together, clamped with slide glasses and fixed with a clip. The sheets were allowed to stand at room temperature (about 25°C) for 24 hours or more, to prepare a sample (non-sterilized) for determination of the peel strength of adhesion.

In addition, the above-mentioned sample was subjected to autoclaving (121°C for 20 minutes) or ethylene oxide gas sterilization (60°C for 3 hours) to prepare a sample for determination of the peel strength of adhesion.

With respect to each of the samples prepared above, the peel strength before and after sterilization was determined by using Autograph (manufactured by Shimadzu Corporation, AGS-50A) (speed = 10 mm/minute, number of samples n = 3). The results are shown in Table 2.

**Table 2**

| Adhesive | Solvent | Before Sterilization | Autoclaving | Ethylene Oxide Gas Sterilization |
|---|---|---|---|---|
| Prep. Ex. 7 | Toluene | 0.66 ± 0.33 | 1.05 ± 0.11 | 0.79 ± 0.07 |
| Prep. Ex. 8 | Toluene | 0.19 ± 0.08 | 1.38 ± 0.10 | 0.53 ± 0.32 |
| Prep. Ex. 9 | Toluene | 1.35 ± 0.66 | 2.09 ± 0.23 | 0.94 ± 0.17 |
| Comp. Ex. 1 | Toluene | 0.05 ± 0.01 | 0.06 ± 0.03 | 0.03 ± 0.01 |
| Prep. Ex. 11 | Tetrahydrofuran | 0.50 ± 0.51 | 0.76 ± 0.23 | 1.21 ± 0.05 |
| Prep. Ex. 12 | Tetrahydrofuran | 0.89 ± 0.21 | 1.06 ± 0.48 | 0.78 ± 0.18 |
| Prep. Ex. 13 | Tetrahydrofuran | 1.05 ± 0.60 | 1.21 ± 0.60 | 1.07 ± 0.31 |
| n = 3 unit (kgf/cm) | | | | |

It is seen from the results of Table 2 above that the adhesives of Preparation Examples 7 to 9 and 11 to 13 have excellent adhesive strength.

### Test Example 2: Changes in Peel Strength of Adhesion Before and After Sterilization

Sheets (thickness: 100 µm) made of a mixture of the hydrogenated block copolymer obtained in Preparation Example 3 and polypropylene (mixing ratio: 70:30), each sheet having a rectangular shape of 1 cm × 2 cm were cut out, and 15 µl of the adhesive obtained in Preparation Examples 8 to 10 or Comparative Example 1 was applied to a half of a side (1 cm²), and the other half was covered with a Teflon sheet having a thickness of 100 µm. Thereafter, the two sheets were pasted together, clamped with slide glasses and fixed with a clip. The sheets were allowed to stand at room temperature (about 25°C) for 24 hours or more, to prepare a sample (non-sterilized) for determination of the peel strength of adhesion.

In addition, the above-mentioned sample was subjected to autoclaving (121°C for 20 minutes) or ethylene oxide gas sterilization (60°C for 3 hours) to prepare a sample for determination of the peel strength of adhesion.

With respect to each of the samples prepared above, the peel strength before and after sterilization was determined by using Autograph (manufactured by Shimadzu Corporation, AGS-50A) (speed = 10 mm/minute, number of samples n = 3). The results are shown in Table 3.

**Table 3**

| Adhesive | Solvent | Before Sterilization | Autoclaving | Ethylene Oxide Gas Sterilization |
|---|---|---|---|---|
| Prep. Ex. 8 | Toluene | 1.90 ± 0.50 | 3.60 ± 0.10 | 2.20 ± 0.10 |
| Prep. Ex. 9 | Toluene | 1.93 ± 0.13 | 2.43 ± 0.26 | 2.00 ± 0.21 |
| Prep. Ex. 10 | Toluene | 0.73 ± 0.19 | 0.71 ± 0.15 | 0.47 ± 0.02 |
| Comp. Ex. 1 | Toluene | 0.05 ± 0.02 | 0.07 ± 0.03 | 0.04 ± 0.01 |
| n = 3 unit (kgf/cm) | | | | |

### Test Example 3: Changes in Peel Strength of Adhesive Before and After Sterilization

In a dried, nitrogen-exchanged pressure vessel, styrene was polymerized using cyclohexane as a solvent and s-butyllithium as a polymerization initiator. Thereafter, isoprene and styrene were sequentially polymerized, to give a block copolymer of styrene-isoprene-styrene form. Further, the block copolymer was hydrogenated in cyclohexane at a hydrogen pressure of 20 kg/cm² in the presence of palladium-carbon as a catalyst, to give a hydrogenated block copolymer. The resulting hydrogenated block copolymer had the content of styrene of 18%, the number-average molecular weight of 85000, the content of vinyl bonds of 7%, and the hydrogenation ratio of 98%.

Sheets (thickness: 100 µm) made of a mixture of the hydrogenated block copolymer obtained above and polypropylene (mixing ratio: 55:45), each sheet having a rectangular shape of 1 cm × 2 cm were cut out, and 15 µl of the adhesive obtained in Preparation Examples 7 to 9 or Comparative Example 1 was applied to a half of a side (1 cm²), and the other half was covered with a Teflon sheet having a thickness of 100 µm. Thereafter, the two sheets were pasted together, clamped with slide glasses and fixed with a clip. The sheets were allowed to stand at room temperature (about 25°C) for 24 hours or more, to prepare a sample (non-sterilized) for determination of the peel strength of adhesion.

In addition, the above-mentioned sample was subjected to autoclaving (121°C for 20 minutes) or ethylene oxide gas sterilization (60°C for 3 hours) to prepare a sample for determination of the peel strength of adhesion.

With respect to each of the samples prepared above, the peel strength before and after sterilization was determined by using Autograph (manufactured by Shimadzu Corporation, AGS-50A) (speed = 10 mm/minute, number of samples n = 3). The results are shown in Table 4.

**Table 4**

| Adhesive | Solvent | Before Sterilization | Autoclaving | Ethylene Oxide Gas Sterilization |
|---|---|---|---|---|
| Prep. Ex. 7 | Toluene | 1.46 ± 0.17 | 1.93 ± 0.30 | 1.44 ± 0.19 |
| Prep. Ex. 8 | Toluene | 1.97 ± 0.14 | 2.72 ± 0.22 | 1.83 ± 0.21 |
| Prep. Ex. 9 | Toluene | 1.53 ± 0.21 | 3.08 ± 0.44 | 1.61 ± 0.25 |
| Comp. Ex. 1 | Toluene | 0.37 ± 0.15 | 0.70 ± 0.19 | 0.32 ± 0.09 |
| n = 3 unit (kgf/cm) | | | | |

### Test Example 4: Changes in Pull-out Strength Before and After Sterilization

A vessel indwelling catheter comprising the following materials was assembled, and a pull-out strength test before and after the sterilization was conducted.
1) Sample:
- catheter: a tube molded product (inner diameter of 0.7 mm × outer diameter of 1.15 mm) made of a mixture of the hydrogenated block copolymer obtained in Preparation Example 3 and polypropylene (mixing ratio: 20:80);
- junction adapter: a tube molded product (inner diameter of 1.2 mm × outer diameter of 2.65 mm) made of a mixture of the hydrogenated block copolymer obtained in Preparation Example 3 and polypropylene (mixing ratio: 20:80); and
- connector: a molded product made of a mixture of the hydrogenated block copolymer obtained in Preparation Example 3 and polypropylene (mixing ratio: 10:90).

2) Adhesion of materials: Using the adhesive obtained in Preparation Example 8, the materials were adhered each to an inserted part of 1 cm in the order of the catheter, the junction adapter and the connector. The resulting product was allowed to stand at room temperature (about 25°C) for 24 hours or more, to prepare a sample for a pull-out test.
3) Sterilization treatment: The above-mentioned sample was subjected to autoclaving (121°C for 20 minutes) or ethylene oxide gas sterilization (60°C for 3 hours).
4) Determination of pull-out strength: With respect to the above-mentioned sample, the pull-out strength before and after the sterilization was determined by using Autograph (manufactured by Shimadzu Corporation, AGS-50A). The pull-out strength was determined by recognizing the catheter and the connector as both ends (speed = 10 mm/minute, number of samples n = 3). The results are shown in Table 5.

**Table 5**

| Adhesive | Solvent | Before Sterilization | Autoclaving | Ethylene Oxide Gas Sterilization |
|---|---|---|---|---|
| Prep. Ex. 8 | Toluene | 1.9 ± 0.1 | 2.4 ± 0.1 | 2.0 ± 0.0 |
| n = 3 unit (kgf) | | | | |

It is seen from the results in Table 5 that the medical device produced by adhering parts with the adhesive of Preparation Example 8 has sufficient pull-out strength.

### INDUSTRIAL APPLICABILITY

According to the process of the present invention, there is provided a medial device with strong adhesive strength at an adhering part, and without lowering adhesive strength even when subjected to sterilization, or even in blood, body fluid, physiological saline and ethanol, which is highly safe because there is no substance eluted into blood, body fluid, physiological saline and ethanol.

## Claims

1. A process for producing a medical device, **characterized by** adhering parts for a medical device comprising an olefinic resin (I) and/or a thermoplastic elastomer (II) with an adhesive comprising a solution prepared by dissolving in an organic solvent a block copolymer or a hydrogenated product thereof, the block copolymer comprising 10 to 40% by weight of a polymer block (A) comprising an aromatic vinyl compound unit; and 90 to 60% by weight of at least one polymer block (B) selected from the group consisting of a polyisoprene block (b1), an isoprene-butadiene polymer block (b2), and a polybutadiene block (b3).

2. The process for producing a medical device according to claim 1, wherein the aromatic vinyl compound unit in the polymer block (A) is styrene unit.

3. The process for producing a medical device according to claim 1 or 2, wherein the polymer block (B) is a polyisoprene block (b11) having a content of 1,2-bond and 3,4-bond of 10% by mol or more.

4. The process for producing a medical device according to claim 1 or 2, wherein the polymer block (B) is an isoprene-butadiene polymer block (b21) comprising 5 to 95% by weight of isoprene unit and 95 to 5% by weight of butadiene unit and having a content of 1,2-bond and 3,4-bond of 20% by mol or more.

5. The process for producing a medical device according to claim 1 or 2, wherein the polymer block (B) is a polybutadiene block (b31) having a content of 1,2-bond of 30% by mol or more.

6. The process for producing a medical device according to any one of claims 1 to 5, wherein the thermoplastic elastomer (II) is at least one block copolymer selected from the group consisting of a block copolymer and a hydrogenated product thereof, the block copolymer comprising a polymer block (C) comprising an aromatic vinyl compound unit and a polymer block (D) comprising a conjugated diene unit, and having a content of the aromatic vinyl compound unit of from 10 to 40% by weight.

7. The process for producing a medical device according to claim 6, wherein the aromatic vinyl compound unit in the polymer block (C) is styrene unit.

8. The process for producing a medical device according to claim 6 or 7, wherein the polymer block (D) is at least one polymer block selected from the group consisting of a polyisoprene block (d1), an isoprene-butadiene polymer block (d2) and a polybutadiene block (d3).

9. The process for producing a medical device according to claim 8, wherein the polymer block (D) is a polyisoprene block (d11) having a content of 1,2-bond and 3,4-bond of 10% by mol or more.

10. The process for producing a medical device according to claim 8, wherein the polymer block (D) is an isoprene-butadiene polymer block (d21) comprising 5 to 95% by weight of isoprene unit and 95 to 5% by weight of butadiene unit and having a content of 1,2-bond and 3,4-bond of 20% by mol or more.

11. The process for producing a medical device according to claim 8, wherein the polymer block (D) is a polybutadiene block (d31) having a content of 1,2-bond of 30% by mol or more.

12. The process for producing a medical device according to any one of claims 6 to 11, **characterized in that** 70% by mol or more of the carbon-carbon double bond in the polymer block (D) is hydrogenated.

13. The process for producing a medical device according to any one of claims 1 to 12, wherein the medical device is one device selected from the group consisting of blood bag, blood tube, infusion solution bag, infusion solution tube, blood circuit, and catheter.

14. A medical device obtainable by the process according to any one of claims 1 to 13.

15. An adhesive comprising a solution prepared by dissolving in an organic solvent a block copolymer or a hydrogenated product thereof, the block copolymer comprising 10 to 40% by weight of a polymer block (A) comprising an aromatic vinyl compound unit; and 90 to 60% by weight of at least one polymer block (B1) selected from the group consisting of a polyisoprene block (b11) having a content of 1,2-bond and 3,4-bond of 10% by mol or more, an isoprene-butadiene polymer block (b21) comprising 5 to 95% by weight of isoprene unit and 95 to 5% by weight of butadiene unit and having a content of 1,2-bond and 3,4-bond of 20% by mol or more, and a polybutadiene block (b31) having a content of 1,2-bond of 30% by mol or more.

16. The adhesive according to claim 15, wherein the aromatic vinyl compound unit in the polymer block (A) is styrene unit.

17. Use of an adhesive of claim 15 or 16, for producing a medical device by adhering parts of a medical device comprising an olefinic resin (I) and/or a thermoplastic elastomer (II).

18. A medical method comprising the step of applying the medical device of claim 14 to a human body.
